# EUROPÄISCHE PATENTSCHRIFT

(11) **EP 3 310 306 B1**
(45) Veröffentlichungstag und Bekanntmachung des Hinweises auf die Patenterteilung: **24.07.2019**
(21) Anmeldenummer: 16733347.5
(22) Anmeldetag: 20.06.2016
(51) Int. Cl.: A61F 5/01, A61F 5/34

(54) **VORRICHTUNG ZUR BEHANDLUNG DES KARPALTUNNELSYNDROMS DER HAND EINER PERSON**
DEVICE FOR TREATING THE CARPAL TUNNEL SYNDROM OF A PERSON'S HAND
DISPOSITIF POUR TRAITER LE SYNDROME DU CANAL CARPIEN DE LA MAIN D'UNE PERSONNE

(30) Priorität: 22.06.2015 DE 202015103260 U
(43) Veröffentlichungstag der Anmeldung: 25.04.2018
(73) Patentinhaber: Curmed GmbH & Co. KG, 78052 Villingen-Schwenningen (DE)
(72) Erfinder: HEITER, Uwe, 78052 Villingen-Schwenningen (DE)
(74) Vertreter: Arat, Dogan
(86) Internationale Anmeldenummer: PCT/EP2016/064149
(87) Internationale Veröffentlichungsnummer: WO 2016/207100

(56) Entgegenhaltungen:
- EP-B1- 2 664 306
- WO-A1-03/017886
- US-A1- 2004 210 169
- US-B2- 6 808 501

## Beschreibung

Die Erfindung betrifft eine Handmanschette für eine Vorrichtung zur Behandlung des Karpaltunnelsyndroms der Hand einer Person gemäß dem Oberbegriff des Patentanspruchs 1 und eine Vorrichtung zur Behandlung des Karpaltunnelsyndroms der Hand einer Person.

Das Karpaltunnelsyndrom bezeichnet ein Kompressionssyndrom des Nervus Medianus im Bereich des Karpaltunnels, der auf der Palmarseite bzw. Handinnenflächenseite des Handgelenks verläuft. Typische Symptome sind auftretende Schmerzen oder Missempfindungen, die von der Hand in den gesamten Arm einstrahlen können. Im fortgeschrittenen Stadium kann es zu einem Muskelschwund im Bereich des Daumenballens, Schwäche beim Zupacken und zu einer Minderung des Tastgefühls kommen. Eine chirurgische Therapie ist verbunden mit den Gefahren und Komplikationen eines operativen Eingriffs.

Es ist bekannt, das Karpaltunnelsyndrom konservativ zu behandeln. Dabei vermag das Tragen spezieller Nachtschienen oder auch das Anlegen von geformten Stützverbänden die Beschwerden zumindest für eine Zeit lang zu beseitigen oder abzumildern. Die erfolgt durch sogenannte Handgelenksorthesen, die insbesondere nachts ein schmerzverursachendes Abknicken des Handgelenkes verhindern. Diese Orthesen oder entsprechende Bandagen reduzieren den Schmerz zeigen aber keinen ausreichenden therapeutischen Effekt und bekämpfen daher nicht die Ursache der Schmerzen, wie es dem BARMER GEK Heil- und Hilfsmittelreport 2012 von Claudia Lemper et al. Vom September 2012 zu entnehmen ist. Derartige Handgelenksorthesen sind beispielsweise aus dem US-Patent US 7,175,603 B1 und den Patentanmeldungen EP 2 060 244 A1, DE 43 26 751 A1 und US 2010/0298750 A1 bekannt.

Eine Alternative zu diesen Handgelenksorthesen sind spezielle Schienen, die den Karpaltunnel der Hand öffnen, indem dieser gedehnt und dadurch die querliegenden Bänder verlängert werden.

Dieser Prozess verringert den Druck auf die Nerven, was die Schmerzen mildert und die Heilung der Entzündung ermöglicht.

Die Offenlegungsschrift WO 03/017885 A1 beschreibt eine automatische Vorrichtung und ein Verfahren zur Behandlung des Karpaltunnelsyndroms, das ein Gehäuse zur Aufnahme der Hand des Patienten mit Druckelementen umfasst, die in Kontakt mit dem Hypothenarbereich bzw. der kleinfingerseitigen Handfläche, dem Thenarbereich bzw. der daumenseitigen Handfläche und dem Dorsalbereich bzw. dem Handrücken der Hand stehen. Bei dem Gehäuse zur Aufnahme der Hand handelt es sich um ein geschlossenes Gehäuse in 0-Form mit einem Loch für den Daumen. Hierdurch ist es ungünstigerweise erforderlich, dass zwei aktive Druckquellen für das erste Druckelement einen Druck auf den Hypothenarbereich und für das zweite Druckelement einen Druck auf den Thenarbereich der Hand aufbringen. Auf der gegenüberliegenden Dorsalseite der Hand sorgt ein Druckpolster für den Gegendruck. Dabei steuert die Steuereinheit die beiden aktiven Druckquellen derart an, dass der Thenar- und der Hypothenarbereich der Hand auseinander und um das dorsal angeordnete Druckpolster gezogen wird. Nur in Folge der beiden palmarseitig wirkenden Kräfte und einer dorsalseitig wirkenden Kraft im geschlossenen Gehäuse wird ein Trennen des Handwurzelknochens der Hand bewirkt. Hierbei muss das Gehäuse sehr genau nach der Größe der zu behandelnden Hand bemessen und daraufhin hergestellt sein, sonst verringert sich der Wirkungsgrad.

Aus der Offenlegungsschrift WO 03/017886 A1, die als nächstliegender Stand der Technik angesehen werden kann, ist eine vergleichbare Vorrichtung und ein Verfahren zur Behandlung des Karpaltunnelsyndroms, das ein Gehäuse zur Aufnahme der Hand des Patienten mit Druckelementen umfasst, die in Kontakt mit dem Hypothenarbereich bzw. der kleinfingerseitigen Handfläche, dem Thenarbereich bzw. der daumenseitigen Handfläche und dem Dorsalbereich bzw. dem Handrücken der Hand stehen, bekannt. Entgegen der vorgenannten Vorrichtung zeigt diese Vorrichtung zwei Gehäuseteile, die miteinander am Rand über ein Scharnier verbunden sind und gegeneinander verschwenkt werden können. Im geschlossenen Zustand umschließen die beiden Gehäuseteile die Hand des Patienten. Diese Vorrichtung erweist sich in der Handhabung als umständlich.

Aus dem US-Patent US 6,146,347 B1 ist eine Vorrichtung und Verfahren zur Behandlung des Karpaltunnelsyndroms bekannt. In der Offenlegungsschrift WO 03/007804 A2 ist eine weitere Vorrichtung und Verfahren zur Behandlung des Karpaltunnelsyndroms mit einem C-förmigen Gehäuse zur Aufnahme der rechten oder linken Hand mit einem Druckelement im dorsalen Abschnitt offenbart. Das Druckelement ist mit einer aktiven Druckquelle verbunden, so dass, wenn die Hand in das Gehäuse eingelegt ist, das Druckelement aktiviert werden kann, um Druck auf der Dorsalseite der Hand auszuüben. Die Vorrichtung enthält starre Abschnitte zur Kontaktierung der Thenar- und Hypothenarbereiche der Hand. Durch die eine abwärtsgerichtete Kraft des Druckelementes und die Hebelwirkung der starren Abschnitte wird eine Dehnung des Handwurzelknochens bewirkt. Hierbei ist es erforderlich, das Gehäuse so zu bemessen und zu formen, dass die Hand des Patienten wirkungsvoll auf genommen werden kann und durch präzise gesteuerte Querstreckung behandelt werden kann. Wegen seiner Konstruktion aus starrem Material muss das Gehäuse deshalb in den unterschiedlichen Größen S (klein), M (mittel) und L (groß) hergestellt werden. Durch Messung der Handbreite wird die für unterschiedliche Patienten geeignete Größe ermittelt. Die Verwendung der richtigen Größe des Gehäuses ist ein entscheidender Faktor für die Erfolgsaussichten der Behandlung. Ist das Gehäuse zu schmal, wird die zu behandelnde Hand gestaucht und kann durch das Druckelement nicht ausreichend durchgedrückt werden. Hierdurch verringert sich der Wirkungsgrad, weil keine ausreichende Dehnung des Karpaltunnels erfolgt. Trotz genauer Anleitung, wie die Messung der Handbreite zu erfolgen hat, ist diese Methode stark anfällig für Falschmessungen. Das wiederum hat einen vergleichsweise hohen Anteil an Rücksendungen bzw. Austausch der Geräte zur Folge. Wie viele Patienten die Behandlung mit einer für sie geeigneten Manschette durchführen, lässt sich nur schwer überprüfen.

In dem Europäischen Patent EP 2 664 306 B1 ist eine Vorrichtung zur Behandlung des Karpaltunnelsyndroms mit einem C-förmigen Gehäuse zur Aufnahme der rechten oder linken Hand mit einem Druckelement im dorsalen Abschnitt offenbart. Diese offene Struktur des C-förmigen Gehäuses zur Aufnahme der rechten oder linken Hand ermöglicht es entgegen den allseits umschließenden Handgelenksorthesen den Karpaltunnel der Hand öffnen, indem dieser gedehnt und dadurch die querliegenden Bänder verlängert werden. Diese Vorrichtung ermöglicht eine differenzierte Behandlung indem in der Handmanschette zwei unterschiedlich starke Einlagen angebracht werden können, die es dem Patienten erlauben, die Größe der Handmanschette anzupassen bzw. zu verkleinern. Der Patient erhält zusammen mit einer einheitlichen, C-förmigen und einstückigen Handmanschette einen Satz von unterschiedlich starken und farblich gestalteten Einlagen. Der Patient muss nicht mehr die für ihn richtige Größe der Handmanschette durch Messung seiner Handbreiten ermitteln. Die mitgelieferten unterschiedlich starken Einlagen ermöglichen es dem Patienten, selbständig die für ihn passende Größe durch Ausprobieren anzupassen und bei Bedarf durch Austauschen/Entfernen der Einlagen zu ändern. Vorteilhaft wird so die Handhabung der Manschette für den Patienten vereinfacht und es wird ihm stets die richtige Größe gegeben. Diese Vorrichtung zeigt demnach durch die Wahl der verschiedenen Einlagen eine für den Patienten schwierige Handhabung.

Der Erfindung liegt daher die Aufgabe zugrunde, eine Vorrichtung zur Behandlung des Karpaltunnelsyndroms und eine Handmanschette für eine Vorrichtung zur Behandlung des Karpaltunnelsyndroms bereitzustellen, deren Handhabung in einer komfortablen und kontrollierten Art und effizienten Weise erfolgen kann.

Diese Aufgabe wird durch eine Handmanschette gemäß Anspruch 1 und eine Vorrichtung gemäß Anspruch 17 gelöst.

Vorteilhafte Ausgestaltungen der Erfindung sind Gegenstand der Unteransprüche.

Die erfindungsgemäße Lösung besteht darin, die Handmanschette für eine Vorrichtung zur Behandlung des Karpaltunnelsyndroms der Hand einer Person, die eine Palmarseite mit Thenar- und Hypothenar-Regionen und eine Dorsalseite gegenüber der Palmarseite aufweist und die im Querschnitt als ein C-Profil ausgebildet ist, aus zwei oder mehreren Manschettenteilen auszubilden. Dabei ermöglicht die einseitig offene Struktur der C-förmigen Handmanschette zur Aufnahme der rechten oder linken Hand entgegen den allseits umschließenden Handgelenksorthesen den Karpaltunnel der Hand öffnen, indem dieser gedehnt und dadurch die querliegenden Bänder verlängert werden und dadurch das Karpaltunnelsyndrom zielgereichtet an der Ursache zu behandeln und nicht nur die Symptome zu bekämpfen. Die Manschettenteile zeigen alle oder zumindest teilweise ein im Querschnitt C-förmiges Profil und sind miteinander mittels wenigstens eines Verbindungselementes zu der im Querschnitt C-profilförmigen Handmanschette verbindbar. Dabei werden die Manschettenteile so miteinander verbunden, dass die verbundene Handmanschette geeignet ist, ein Widerlager für ein eingebrachtes Druckpolster zu bilden und den Karpaltunnel der Hand öffnen, indem dieser gedehnt und dadurch die querliegenden Bänder verlängert werden und dadurch das Karpaltunnelsyndrom zielgereichtet an der Ursache behandelt und nicht nur die Symptome zu bekämpfet werden. Auch werden die Manschettenteile so miteinander verbunden, dass sie eine patentientengeeignete Manschettenöffnung aufweisen und abhängig von der Verbindung einen entsprechenden, patientengeeigneten, differenzierten Querschnitt aufweisen. Dadurch wird es insbesondere möglich, dass durch das Verbinden der C-förmigen Manschettenteile eine unterschiedliche Öffnung mit unterschiedlichem Querschnitt zur Aufnahme der Hand einer Person, die an dem Karpaltunnelsyndrom leidet, geschaffen wird. Durch diese bevorzugte Möglichkeit der Einstellung mit Hilfe des Verbindungselementes gelingt es auf sehr einfache, verlässliche und komfortable Weise, eine effiziente Behandlung des Karpaltunnelsyndroms eines Patienten zu ermöglichen, und dabei die Handmanschette individuell auf die Bedürfnisse des Patienten anzupassen. Durch die mehrteilige Ausbildung der Manschettenteile der Handmanschette in Verbindung mit der Möglichkeit der individuellen Anpassung des Querschnitts der zugehörigen Öffnung für die Hand mit Hilfe des Verbindungselementes oder der Verbindungselemente wird es sogar möglich, dass häufig auf das Vorsehen einer Einlage in die Handmanschette entsprechend dem Stand der Technik verzichtet werden kann.

Eine besonders einfach zu handzuhabende Handmanschette zur Behandlung des Karpaltunnelsyndroms weist zwei Manschettenteile auf, die im Querschnitt C-förmig ausgebildet sind und durch das erfindungsgemäße Verbindungselement zu einer im Querschnitt C-förmigen erfindungsgemäßen Handmanschette verbindbar sind. Diese zwei Manschettenteile erweisen sich als besonders vorteilhaft, da dadurch die Akzeptanz und Handhabbarkeit des Patienten hinsichtlich der Einstellung und Verbindung der Manschettenteile zu der Handmanschette in besonders vorteilhafter Weise gegeben ist.

Als besonders vorteilhaft hat es sich erwiesen, das Verbindungselement so auszubilden, dass die verbundenen Manschettenteile gegeneinander verschiebbar und/oder verschwenkbar sind und dadurch eine Anpassung der Breite der Manschettenöffnung der C-förmigen Handmanschette und damit eine Vergrößerung oder Verkleinerung der Querschnittsöffnung der Handmanschette zur individuellen Anpassung an die Bedürfnisse des Patienten gegeben ist. Durch das Vorsehen einer Anpassung der Verbindung durch Verschieben, was sowohl in Form einer Verschiebung entlang einer linearen Geraden oder einer leicht gekrümmten, insbesondere kreisförmig gekrümmten Linie erfolgt, ist eine sehr einfache und sichere Anpassung und Handhabung der Handmanschette im Hinblick auf die Anpassung an die individuellen Bedürfnisse geschaffen. Entsprechend gilt das auch für eine Anpassung der Breite durch eine Verschwenkbewegung, bei der um eine feste oder verschiebliche Achse an einem Manschettenteil die beiden Manschettenteile gegeneinander verschwenkt und die Breite der der Manschettenöffnung verändert und an die Bedürfnisse des Patienten angepasst werden kann.

Insbesondere lässt sich dadurch eine Vielzahl von Zwischenpositionen für die individuelle Anpassung nutzen. Dabei werden einzelne Positionen insbesondere durch Rastelemente markiert, so dass ein ungewolltes Verlassen der gewünschten Verschiebe-, Verschwenkungs- bzw. Verbindungsposition behindert wird, ohne dass eine einfache, komfortable und sichere Anpassung ausgeschlossen oder verhindert wird.

Auch hat es sich bewährt, neben einer Mehrzahl an bevorzugten, vorgesehenen Zwischenpositionen, die Möglichkeit zu schaffen, jede mögliche Zwischenposition aus den kontinuierlich verbundenen Zwischenpositionen des Verschiebungsweges und/oder der Verschwenkungspositionen auszuwählen und in dieser Position diese durch das verschieblich und/oder verschwenkbar ausgebildete Verbindungselement zu verbinden und diese Position zu halten.

Die Anordnung eines oder mehrerer Verbindungselemente in Abschnitten der Manschettenteile, die sich wechselseitig überlappen, hat sich besonders bewährt, da dort auch eine einfache und effiziente Verbindung mit Hilfe des oder der Verbindungselemente geschaffen ist. Dies ist umso effizienter, je weniger gekrümmt die überlappenden Abschnitte mit den betreffenden Verbindungselementen ausgebildet sind. Als besonders vorteilhaft haben sich plane überlappende Flächen erwiesen, da diese besonders einfach und verlässlich aufeinander gleitend verschoben werden können und dadurch eine sehr verlässliche Verbindung mit Hilfe des oder der Verbindungselemente geschaffen ist.

Alternativ zu den planen überlappenden Abschnitten haben sich auch zylindrisch gekrümmte Flächen herausgestellt, die mittels einer Verschwenkbewegung gegeneinander verschoben werden können und dadurch mit Hilfe eines Verbindungselementes sicher verbunden werden können. Gerade bei gleichen oder wenig unterschiedlichen Zylinderradien ist dies sehr verlässlich möglich. Die zylindrisch gekrümmten Flächen der überlappenden Abschnitte ermöglichen eine erfindungsgemäße Handmanschette mit C-förmigem Querschnitt, die sich als besonders gut handhabbar erweist. Auch zeigen geringe Abweichungen von einem konstanten Zylinderradius keine wesentliche Einschränkung der positiven Wirkung. Die Handhabung eines Patienten zur Adaption an die individuellen Bedürfnisse ist bei den planen oder auch leicht zylindrisch gekrümmten, überlappenden Abschnitten in besonderem Maße gewährleistet.

Soll die erfindungsgemäße Vorrichtung ein Druckpolster aufweisen, welches in die Handmanschette eingebracht wird, so hat es sich besonders bewährt, wenigstens ein Verbindungselement der erfindungsgemäßen Handmanschette mit mehreren Öffnungen zur Aufnahme eines Schlauches zur Befüllung oder Entnahme eines Fluides für das in die Handmanschette eingebrachte Druckpolster vorzusehen. Dabei ist wenigstens eine Öffnung im Bereich des Verbindungselementes als Langloch ausgebildet, so dass die beiden im Bereich des Verbindungselementes überlappenden Manschettenteile gegeneinander verschoben werden können und dadurch erfindungsgemäß die Breite der Manschettenöffnung bzw. der Querschnitt der Handmanschette anpassbar gestaltet werden kann, ohne dass ein in die überlappenden Öffnungen des Verbindungselementes eingebrachter Schlauch abgeschert oder eingeklemmt wird. Durch das Langloch ist das erfindungsgemäße vorgesehene Verschieben der Manschettenteile durch das verschieblich ausgebildete Verbindungselement in besonders vorteilhafter Weise gewährleistet. Durch die gezielte Wahl der Länge des Langlochs lässt sich vorteilhafterweise auch das Maß der individuellen Einstellbarkeit des möglichen Verschiebeweges und/oder des Verschwenkungsbereiches der Manschettenteile gegeneinander und damit das Maß der Breite der Manschettenöffnung definieren und in diesem Bereich variieren. Dadurch wird die Handhabung der erfindungsgemäßen Handmanschette auf vorteilhafte Weise beschränkt und dadurch eine Fehlhandhabung, z. B. durch unsachgemäßes Verbinden der Manschettenteile mit Hilfe des Verbindungselementes ausgeschlossen.

Als besonders vorteilhaft hat es sich erwiesen, Manschettenteile mit Führungselementen zur Festlegung der möglichen Verschiebung und/oder Verschwenkung der Manschettenteile gegeneinander zu versehen. Dadurch ist ein definiertes Verschieben und/oder Verschwenken der Manschettenteile in einem begrenzten räumlichen Bereich gegeneinander festgelegt und auch durch einen wenig versierten Patienten gewährleistet und dadurch eine Fehlhandhabung weitgehend ausgeschlossen.

Als besonders bevorzugte Führungselemente haben sich Verbindungselemente wie Nut-Feder-Verbindungen, Kulissenführungen und/oder Randführungen erwiesen. Die Führungselemente sind aber nicht auf diese spezifisch genannten Führungselemente beschränkt. Neben der Möglichkeit, ein einziges zusammenhängendes Paar an Führungselementen vorzusehen, hat es sich als besonders vorteilhaft erwiesen, zwei oder mehrere, insbesondere parallel zueinander verlaufende Paare an insbesondere gleichartigen Führungselementen vorzusehen. Durch diese gelingt es, eine besonders verlässliche Führung während des Verschiebungs- und/oder Verschwenkungsvorganges und damit eine Möglichkeit der individuellen Anpassung an die Bedürfnisse des einzelnen Patienten zu gewährleisten, indem das Risiko eines Verkantens und damit einer Blockade der Manschettenteile während des individuellen Anpassens weitgehend verhindert ist.

Gerade das Vorsehen von einem Paar an Führungselementen in der Art einer Nut-Feder-Verbindung, bei der die Feder entlang der Nut verschieblich geführt wird und dadurch ein laterales Herausgleiten der Feder aus der Nut verhindert wird, zeigt die vorteilhafte Wirkung. In entsprechender Weise hat es sich bewährt, eine Kulissenführung aus einem Paar von Führungselementen zu realisieren, bei der ein Kulissenstein in einer Kulisse geführt wird und dadurch eine Zwangsführung entlang der Bahn der Kulisse erreicht wird. Alternativ hierzu hat es sich auch bewährt, eine sogenannte Randführung vorzusehen, bei der zwei gegenüberliegende Ränder eines Manschettenteils seitlich durch Führungselemente geführt werden. Diese sind insbesondere in Form von Stiften oder Erhebungen, welche sich von dem korrespondierenden Manschettenteil erheben, ausgebildet. Sie verhindern ein laterales Versetzen der Manschettenteile gegeneinander, indem diese Führungselemente den Rand in der Art eines seitlichen, lateralen Anschlages zwangsführen und dadurch eine Längsführung gewährleisten. Zusätzlich kann die Randführung durch Vorsehen von Höhenanschlägen auch ein ungewolltes vertikales herausgleiten eines Manschettenteils aus dem verbundenen anderen Manschettenteil verhindern. Diese Führungselemente haben sich besonders bewährt, da sie eine sehr definierte Möglichkeit der individuellen Positionierung der Manschettenteile gegeneinander und damit eine Anpassung der Breite der Manschettenöffnung auf einfache und effiziente Weise gewährleisten. Eine Fehlhandhabung ist dadurch wenig wahrscheinlich.

Bei einer besonders vorteilhaften Ausbildung der erfindungsgemäßen Handmanschette sind Führungselemente als eine oder mehrere Kulissenführungen ausgebildet, in denen eine oder mehrere Kulissensteine mit Hinterschnitt in eine oder mehrere Kulissenlaufbahnen eingreifen, wobei die Kulissensteine mit ihrem Profil dem Profil der zugeordneten Kulissenlaufbahn entsprechen. Damit ist neben der lateralen Führung ein unerwünschtes vertikales Herausgleiten der Kulissensteine aus der Kulissenlaufbahn, die gemeinsam ein Führungselement bilden, eingeschränkt oder unmöglich gemacht. Durch die unveränderbare Gestalt eines Kulissensteines und der einheitlichen Gestaltung der Kulissenlaufbahn wird vorteilhaft sichergestellt, dass die über die Kulissenführung verbundenen Manschettenteile untrennbar sind und damit die Funktionsfähigkeit der Handmanschette auch unter extremen Bedingungen immer gewährleistet ist.

Erfindungsgemäß wird zusätzlich in der Kulissenlaufbahn ein Trennbereich vorgesehen, der eine Entnahme und/oder ein Einbringen des profilierten Kulissensteins aus der oder in die profilierte Kulissenlaufbahn ermöglicht. Dies wird insbesondere dadurch erreicht, dass die profilierte Kulissenlaufbahn im Trennbereich nicht mehr dem Profil des Kulissensteins entspricht sondern vielmehr eine Ausnehmung aufweist, durch die der Kulissenstein aus der Kulissenlaufbahn entnommen oder eingefügt werden kann. Durch dieses ist ein vollständiges Trennen bzw. Zusammenführen der Manschettenteile mit dem als Kulissenstein mit zugeordneter Kulissenlaufbahn ausgebildeten Führungselement als Teil des Verbindungselements gegeben. Dies bewirkt einerseits eine gute Handhabung, die sich durch einfache und sichere Führung auszeichnet, sowie eine besonders gute Herstellbarkeit der einzelnen Teile als Teil der Handmanschette.

Der Trennbereich wird dabei bevorzugt so ausgebildet, dass er in seiner Form dem Kulissenstein entspricht, wobei die Größe des Trennbereiches bevorzugt so gewählt ist, dass die Ausnehmung etwas größer gewählt ist als die Größe des Kulissensteins. Zusätzlich wird bevorzugt die Ausnehmung des Trennbereichs in ihrer Gestalt so gewählt, dass die der Gestalt des Kulissensteins entspricht und dadurch eine Vorzugsrichtung beim Einbringen bzw. Entnehmen des Kulissensteins aus der Kulissenlaufbahn und damit eine definierte Orientierung der beiden Manschettenteile beim Verbinden oder Trennen und eine sichere und korrekte Handhabung gegeben ist. Dies wird insbesondere dadurch erreicht, dass eine drehinvariante Gestalt des Kulissensteins oder zumindest eine Gestalt des Kulissensteins gewählt wird, die wenige Vorzugsrichtungen, also Orientierungen für das Trennen oder Verbinden und damit einen Kulissenstein mit wenigen Drehinvarianten aufweist. Dies führt zu einer sehr sicheren Handhabung, indem das Verbinden oder Trennen der Manschettenteile nur in der gewünschten, korrekten und vorgegebenen Orientierung der Manschettenteile zueinander ermöglicht ist.

Es hat sich als besonders vorteilhaft erwiesen, ein oder mehrere Manschettenteile mit einer Begrenzung der Verschiebung und/oder der Verschwenkung, insbesondere durch einen Anschlag und/oder durch ein Kulisssenlaufbahnende einer Kulissenführung zu versehen. Durch diese Begrenzung der Verschiebung und/oder der Verschwenkung gelingt es, die Handhabung der Handmanschette und damit den Verstellbereich der Manschettenteile gegeneinander zu begrenzen und dadurch die funktionell und anatomisch sinnvollen Breiten der Manschettenöffnung zu definieren und nicht sinnvolle Manschettenöffnung durch die Begrenzung auszuschließen.

Daneben hat es sich auch bewährt, das Verbindungselement der gegeneinander verschiebbaren und/oder verschwenkbaren Manschettenteile mit einer Skala zur Darstellung eines Maßes der Verschiebung und/oder der Verschwenkung bzw. der Breite der Manschettenöffnung der Handmanschette zu versehen. Dadurch wird es auf besonders vorteilhafte Weise ermöglicht, eine erfolgreiche und bewährte Einstellung der Breite der Manschettenöffnung, z. B. nach einer Reinigung, Desinfektion, Sterilisierung oder nach einer Verstellung und Anpassung für einen anderen Patienten wieder korrekt einzustellen und für den entsprechenden Patienten wieder zugänglich zu machen. Auch lässt sich durch diese Art und Weise eine Dokumentation der Behandlung, insbesondere bei progressiven Therapien mit veränderlichen Breiten der Manschettenöffnung ermöglichen und dadurch die Behandlungsmethode patientenspezifisch anpassen und optimieren.

Dabei wird die Skala bevorzugt parallel zu einer oder mehreren Führungsbahnen angeordnet und insbesondere der Rand der Überlappungsbereiche mit einer Markierung versehen, die auf einem oder an einem der beiden überlappenden Manschettenteile angeordnet ist und als Zeiger wirkt. Der Zeiger bzw. die Markierung wirkt mit der nicht überlappten und damit sichtbaren Skala zusammen und repräsentiert die Position und damit ein Maß für die Breite der Manschettenöffnung. Diese Art der Skala, insbesondere in Verbindung mit der Markierung auf oder an dem anderen Manschettenteil, ermöglicht eine besonders verlässliche Ablesung bzw. Einstellung der Breite der Manschettenöffnung und damit eine sehr sichere und verlässliche therapeutische Anwendung der erfindungsgemäßen Handmanschette zur Behandlung des Karpaltunnelsyndroms einer Hand.

Eine andere bevorzugte Weiterbildung der Erfindung zeigt ein oder mehrere Fixierelemente, das oder die in oder an einem Verbindungselement angeordnet sind. Durch das oder die Fixierelemente ist es möglich, die Position der Manschettenteile in verbundenem Zustand festzulegen und die eingestellte Breite der Manschettenöffnung zu fixieren. Durch die Anordnung des oder der Fixierelemente in einem Verbindungselement kann diese Fixierung auf besonders verlässliche Art und Weise gewährleistet werden und dadurch die Handhabung sehr sicher gestaltet werden. Als beispielhaftes Fixierelement haben sich Schraube-Mutter-Verbindungen, Magnetverbindungen, Klebeverbindungen, Formschlussverbindungen z.B. als Rastverbindungen oder z.B. als Klettverbindungen (z.B. mittels einem Klettband) und/oder Klemmverbindungen, insbesondere mittels Kniehebelklemmelementen, oder eine Kombination davon bewährt.

Vorzugsweise sind das oder die Fixierelemente im Bereich der Führungselemente in einem Verbindungselement angeordnet, so dass einerseits die Einstellung der Breite der Manschettenöffnung verlässlich mit Hilfe der Führungselemente vorgenommen werden kann, die insbesondere in Form einer Verschiebung und/oder Verschwenkung erfolgt, und andererseits wird mit Hilfe der Fixierelemente, die insbesondere mit den Führungselementen zusammenwirken, eine Fixierung gewährleistet.

Eine besonders bevorzugte Fixierung erfolgt mit Hilfe einer Mutter-Schraube-Verbindung, die in einem Führungselement in der Art eines Längsloches angeordnet ist. Mittels der Schraube wird eine verlässliche Führung entlang des als Langloch ausgebildeten Führungselementes bewirkt. Zum Anderen wird durch das Andrehen der Mutter auf der Schraube eine Fixierung bewirkt, indem die Manschettenteile mit den Führungselementen in dem Verbindungselement aufeinander gepresst und damit gegeneinander fixiert werden.

Durch ein alternativ besonders bevorzugtes Fixierelement mittels einer Kniehebelverbindung lässt sich ein einfaches Lösen durch Öffnen des Kniehebels und Verschieben und/oder Verschwenken der Manschettenteile gegeneinander gewährleisten und durch Anspannen des Kniehebels ein Verspannen der Manschettenteile und damit Fixieren dieser Teile gegeneinander bewirken. Eine solche Kniehebelverbindung zeigt einen Hebelarm, an dem an einem Ende ein zylinderförmiges Endstück angeordnet ist, über dessen Umfang sich der Radius des zylinderförmigen Endstücks vergrößert. Dieser Kniehebel ist am Ende eines Bolzens befestigt und lässt sich gegenüber dem Bolzenende verschwenken. Durch das Verschwenken lässt sich der Abstand zwischen dem freien Ende des Bolzens und dem zylinderartigen Endstücks des Kniehebels variieren, entweder vergrößern oder verkleinern. Hierdurch lässt sich ein Lösen oder Verspannen durch Erweitern oder Verkürzen des freien Abstandes zum freien Ende (zylinderförmiges Endstück) und damit des Bereiches, in dem die Manschettenteile im Verbindungsbereich der Verbindungselemente angeordnet sind, anpassen und diese lösen oder gegeneinander fixieren. Gerade diese Art hat sich als besonders vorteilhaft handhabbar erwiesen.

Auch eine Fixierung mittels Magnetverbindung, Klebe- oder Formschlussverbindung hat sich als unterschiedlich vorteilhaft erwiesen, da sie einerseits einfach zu lösen und wieder zu fixieren sind und andererseits bei Bedarf einmalig an die Bedürfnisse eines einzigen Menschen anpassbar sind und danach nicht mehr verändert werden können und damit eine verlässliche Beständigkeit aufweisen. Je nach Bedarf wird das geeignete Fixierelement als einzelnes oder als Kombination mehrerer Fixierelemente gewählt.

Eine besonders bevorzugte Handmanschette zeigt mehrere als Kulissenführungen ausgebildete Führungselemente mit einem oder mehreren Kulissensteinen mit Hinterschnitt und mit einer oder mehreren Kulissenlaufbahnen, die mit einem an den oder die Kulissensteine angepassten Profil versehen sind. Im Zwischenbereich zwischen zwei Kulissenführungen ist wenigstens ein Fixierelement angeordnet. Dadurch ist eine sehr sichere Führung durch das Umschließen des bzw. der Fixierelemente durch Führungselemente und Fixierung der Manschettenteile bei unterschiedlichen Breiten gewährleitet.

Besonders bevorzugt wird im Zwischenbereich eine federnde Lasche angeordnet, die mit einem Taster der Handmanschette zusammenwirkt, mittels dem die federnde Lausche federnd ausgelenkt werden kann und dadurch das Fixierelement im Zwischenbereich gelöst und dadurch die Manschettenteile gegeneinander, geführt durch die Führungselemente, verschoben werden können. Die federnde Lasche wird dabei bevorzugt durch Schlitze in einen Schenkel eines Manschettenteils so gebildet, dass der Bereich zwischen den Schlitzen, der bevorzugt im Zentralbereich der Handmanschette angeordnet ist, federnd bewegt bzw. mit Hilfe des Tasters ausgelenkt werden kann. Vorzugsweise wird durch das Auslenken der federnden Lasche die eine oder mehreren Rastverbindungen, die die Fixierelemente repräsentieren, aus dem Eingriff bzw. Formschluss gebracht, so dass ein freies Bewegen der Manschettenteile gegeneinander zur Einstellung der Breite der Handmanschette, ermöglicht ist. Durch Entlasten der Taste federt die federnde Lasche zurück und damit greift die Rastverbindung wieder formschlüssig ineinander, so dass die Breite der Handmanschette und damit die Relativposition der Manschettenteile zueinander fixiert sind. Durch diese Ausbildung ist eine einfache und sehr sichere Handhabung der erfindungsgemäßen Handmanschette gewährleistet.

Dabei ist die Taste bevorzugt im Bereich einer als Langloch ausgebildeten Öffnung in einem Schenkel des Manschettenteils so angeordnet, dass die Taste von außen durch einen Benutzer betätigt werden kann und eine Verschiebung und/oder Verschwenkung der Manschettenteile so erfolgen kann, dass die Taste über den Verschiebe- und/oder Verschwenkweg, der der Länge des Langlochs entspricht, betätigt werden kann. Die Taste kann dabei als separates Teil realisiert sein oder auch als ein Teil, das mit der federnden Lasche insbesondere einstückig verbunden ist.

Dabei wird die Taste bevorzugt so ausgebildet, dass sie sich durch das Langloch im Bereich der federnden Lasche hindurch erstreckt und bevorzugt dieses überragt. Durch diese Erstreckung gelingt es, eine zusätzliche Führung zu realisieren, die ein zusätzliches Führungselement bildet und dadurch eine sehr verlässliche Führung für den Vorgang des Verschiebens und/oder des Versschenkens der Manschettenteile gegeneinander gewährleistet.

Es hat sich dabei besonders bewährt, den Schlauch zur Zuführung oder Entnahme eines Fluides in das Druckpolster im Bereich dieser erhabenen Taste insbesondere durch den Randbereich der Taste zu führen und dadurch den Schlauch vor Beschädigung insbesondere durch Abscherung zu schützen. Der Taster ist dabei bevorzugt flächig so groß ausgebildet und insbesondere mit Hilfe einer Mulde so geformt, dass eine Betätigung mit Hilfe eines Daumens sehr sicher gewährleistet ist. Als besonders vorteilhaft hat es sich erwiesen, die Manschettenteile lösbar voneinander und damit trennbar voneinander auszubilden und mit Hilfe des oder der Verbindungselemente zu verbinden. Durch diese Art der lösbaren Verbindung der Manschettenteile lässt sich das einzelne Manschettenteil besonders einfach herstellen, besonders einfach reinigen und bei Bedarf auch austauschen bzw. ersetzen. Dadurch ist eine sehr verlässliche Handhabung und Anpassungsmöglichkeit der Handmanschette an die individuellen Gegebenheiten gegeben, insbesondere wenn die gewöhnlichen Anpassungsmöglichkeiten durch Verstellen bzw. Verschieben bzw. Verschwenken nicht ausreichen. Beispielsweise lässt sich durch Ersatz eines oder mehrerer Manschettenteile der Handmanschette mit einem veränderten Verbindungselement oder mehreren Verbindungselementen der Spielraum für eine Anpassung verändern und dadurch die Breite der Manschettenöffnung bzw. die Öffnung der Handmanschette an die erheblich unterschiedlichen individuellen Bedürfnisse verschiedener Patienten anpassen.

Eine besonders vorteilhafte, erfindungsgemäße Handmanschette zeigt zwei oder mehrere Manschettenteile mit C-förmigem Querschnitt mit unterschiedlich langen Schenkeln. Im verbundenen Zustand zeigen die Manschettenteile erfindungsgemäß einen Überlapp der längeren Schenkel und bilden ein Verbindungselement, das die Möglichkeit schafft, die Breite der Manschettenöffnung, die durch kürzere Schenkel begrenzt ist, einzustellen und mit Hilfe eines oder mehrerer Fixierelemente so zu fixieren, dass die Breite während der Anwendung unverändert bleibt. Dabei werden die Manschettenteile durch das Fixierelement oder die Fixierelemente gegeneinander in ihrer Position fixiert. Dies erfolgt vorzugsweise in Form einer lösbaren fixierenden Verbindung, beispielsweise mit Hilfe einer der mehreren Schraube-Mutter-Verbindungen oder einem oder mehreren der anderen der vorgenannten Fixierelemente. Durch die unterschiedlich lang ausgebildeten Schenkel der C-förmigen Manschettenteile ist eine gut handhabbare Handmanschette gegeben, die bei guter Einstellbarkeit und auch guter Effizienz bei der Anwendung ein gutes Einbringen der Hand in die Handmanschette und ein sehr vorteilhaftes Aufbringen der Kraft zur Behandlung des Karpaltunnelsyndroms ermöglicht.

Es hat sich als besonders vorteilhaft erwiesen, im Zentralbereich der Handmanschette, also in Längsrichtung der Handmanschette im Mittelbereich, eine Verstärkung vorzusehen, die wenigstens ein Fixierelement z.B. ein Rastelement, ein Griffelement zur Handhabung und/oder Positionierung der Manschettenteile gegeneinander, ein Führungselement, und/oder ein Loch zur Aufnahme eines Schlauches zur Befüllung oder Entnahme eines Fluides für ein in die Handmanschette einbringbares Druckpolster zeigt. Durch das Vorsehen dieser Verstärkung können diese Elemente besonders verlässlich auch mit geringem Abstand zueinander angeordnet werden, ohne dass wesentliche Einschränkung der Verstellbarkeit oder Fixierbarkeit der Manschettenteile stattfinden. Durch diese Verstärkung und das Anordnen der genannten Elemente im Bereich der Verstärkung ist sichergestellt, dass die für die Behandlung des Karpaltunnelsyndroms notwendige Krafteinwirkung auf die Hand des Patienten in ausreichendem Maße sichergestellt ist, auch wenn zusätzliche potenziell schwächende Elemente in den Manschettenteilen vorgesehen sind.

Mit Hilfe des vorgesehenen Griffelementes im Zentralbereich lassen sich die Handhabung der Handmanschette als Ganzes oder auch die Handhabung der einzelnen Manschettenteile und damit insbesondere eine relative Positionierung der Manschettenteile zueinander und damit eine Einstellung der Breite der Manschettenöffnung abhängig von den individuellen Bedürfnissen des Patienten auf besonders einfache und sichere Weise ermöglichen. Dies umso mehr, da die Handhabung und Einstellung der erfindungsgemäßen Handmanschette bzw. der Vorrichtung zur Behandlung des Karpaltunnelsyndroms durch den Patienten nur und ausschließlich mit der nicht aktuell zu behandelten Hand erfolgt. Dies wird gerade durch das Vorsehen einer derartigen Verstärkung im Zentralbereich in Verbindung mit die Handhabung erleichternden Zusatzelementen in diesem Verstärkungsbereich gewährleistet.

Darüber hinaus ist es vorteilhaft, die erfindungsgemäße Handmanschette im Bereich der Ränder der Manschettenteile abgerundet auszubilden, wodurch eine unerwünschte punktuelle Belastung der Hand im Randbereich verhindert oder deutlich eingeschränkt werden kann. Eine unerwünschte Krafteinwirkung durch die Ränder der Manschettenteile auf die Hand des Patienten kann dadurch möglichst vermieden werden.

Die Erfindung betrifft darüber hinaus eine Vorrichtung zur Behandlung des Karpaltunnelsyndroms der Hand einer Person, mit einer Handmanschette, wie sie zuvor beschrieben ist, und bevorzugt einem Druckpolster, das in der Handmanschette eingebracht ist und das im Zusammenwirken mit den anderen Teilen der Vorrichtung eine Vergrößerung des Karpaltunnels bewirken kann. Diese Vorrichtung zeigt im Zusammenspiel der erfindungsgemäßen Handmanschette mit wenigstens einem Druckpolster eine besonders verlässliche und sichere Handhabung und ermöglicht dadurch eine gute und verlässliche Behandlung des Karpaltunnelsyndroms der Hand eines Patienten.

Die Erfindung ist nachfolgend anhand bevorzugter Ausführungsbeispiele unter Bezugnahme auf die Abbildungen beispielhaft erläutert. Die Erfindung ist nicht auf diese bevorzugten Ausführungsbeispiele beschränkt.

Dabei zeigt schematisch:
- Fig. 1: eine beispielhafte Vorrichtung zur Behandlung des Karpaltunnelsyndroms mit geringer Breite der Manschettenöffnung in einer perspektivischen Ansicht,
- Fig. 2: die Vorrichtung aus Fig. 1 mit vergrößerter Breite der Manschettenöffnung in einer perspektivischen Ansicht,
- Fig. 3: die Vorrichtung aus Fig. 1 während des Einstellens der Breite der Manschettenöffnung in einer perspektivischen Ansicht,
- Fig. 4: die Vorrichtung aus Fig. 1 in einem Querschnitt durch die Vorrichtung gemäß Fig. 3 im Bereich der Fixierelemente,
- Fig. 5: die Vorrichtung aus Fig. 1 in einer horizontalen Schnittdarstellung durch den Überlappbereich der Manschettenteile,
- Fig. 6: eine andere, beispielhafte Vorrichtung zur Behandlung des Karpaltunnelsyndroms mit großer Breite der Manschettenöffnung in einer perspektivischen Ansicht,
- Fig. 7: die Vorrichtung aus Fig. 6 in einer perspektivischen, anderen Ansicht,
- Fig. 8: die Vorrichtung aus Fig. 6 in einer perspektivischen, anderen Ansicht von innen nach außen und
- Fig. 9: die Vorrichtung aus Fig. 6 mit geringer Breite in einer Schnittdarstellung durch den Bereich eines Fixierelementes.

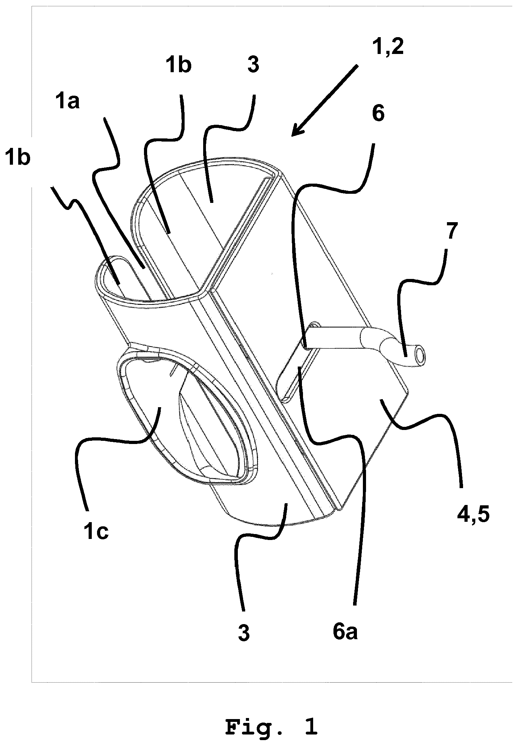

In Fig. 1 ist eine Vorrichtung 2 zur Behandlung des Karpaltunnelsyndroms der Hand einer Person mit einer Handmanschette 1 mit zwei Manschettenteilen 3 dargestellt. Die zwei Manschettenteile 3 haben einen C-förmigen Querschnitt. Sie überlappen sich in einem Abschnitt 5, in dem sich plane Flächen zumindest partiell überlappen. Dieser Abschnitt 5 bildet das Verbindungselement 4 der beiden Manschettenteile. Beide planen Flächen liegen partiell flächig aufeinander. In ihrem Zentralbereich, also in Längsrichtung in der Mitte der Manschettenteile befinden sich im Bereich des Abschnittes 5 Öffnungen 6 und 6a, wobei eine der beiden Öffnungen 6 und 6a als Langloch 6a ausgebildet ist. Durch diese beiden Öffnungen 6, 6a ist von der Innenseite der Handmanschette 1 ein Schlauch 7 eingeführt, mit dessen Hilfe ein in die Handmanschette 1 einbringbares Druckpolster 7a durch ein Fluid befüllt oder entleert werden kann.

Ein Manschettenteil 3 zeigt eine Daumenöffnung 1c. Die Handmanschette 1 zeigt eine Handöffnung 1b, durch die die Hand des Patienten in dorsale, also in Längsrichtung, eingebracht werden kann, bis der Daumen durch die Daumenöffnung 1c hindurch kragt. Die beiden Manschettenteile 3 zeigen mit ihren einen Schenkeln 3a einen Überlapp 5 und sind mit ihren anderen Schenkeln 3b voneinander so beabstandet, dass sie eine Manschettenöffnung 1a bilden. Die Breite der Manschettenöffnung 1a ist abhängig von dem Maß des Überlapps des Abschnittes 5 des Verbindungselementes 4 der beiden Manschettenteile 3 der Handmanschette 1. Die beiden im Querschnitt C-förmigen Manschettenteile 3 werden über das Verbindungselement 4 miteinander verbunden und zeigen eine solche Steifigkeit, so dass die Breite der Manschettenöffnung 1a passend für den jeweiligen Patienten ist und ein solcher mechanischer Druck erzeugt wird, der den Handrücken (auf Dorsalseite) durchdrückt und gegen die, der Handinnenfläche (Palmarseite) zugewandte, offene Seite der Handmanschette drückt. Die durch den Druck erzeugte Hebelwirkung, die gegebenenfalls durch ein Druckpolster 7a verstärkt wird, bewirkt eine Dehnung des auf der Handinnenfläche verlaufenden Karpaltunnels. Die Dehnung des Karpaltunnels vermindert den Druck auf die darin verlaufenden Nervenbahnen und lindert die Symptome des Karpaltunnelsyndroms, was eine sehr wirkungsvolle Behandlungsweise darstellt. Diese Dehnung wird durch die einseitig offene C-förmige Handmanschette erst im besonderen Maße wirksam ermöglicht.

Erfindungsgemäß werden die beiden Manschettenteile 3 so gegeneinander verschoben und miteinander mit Hilfe des Verbindungselementes 4 verbunden, dass die gewünschte Breite der Manschettenöffnung 1a festgelegt ist, welche für den jeweiligen Patienten eine wirkungsvolle Therapie gegen das Karpaltunnelsyndrom ermöglicht.

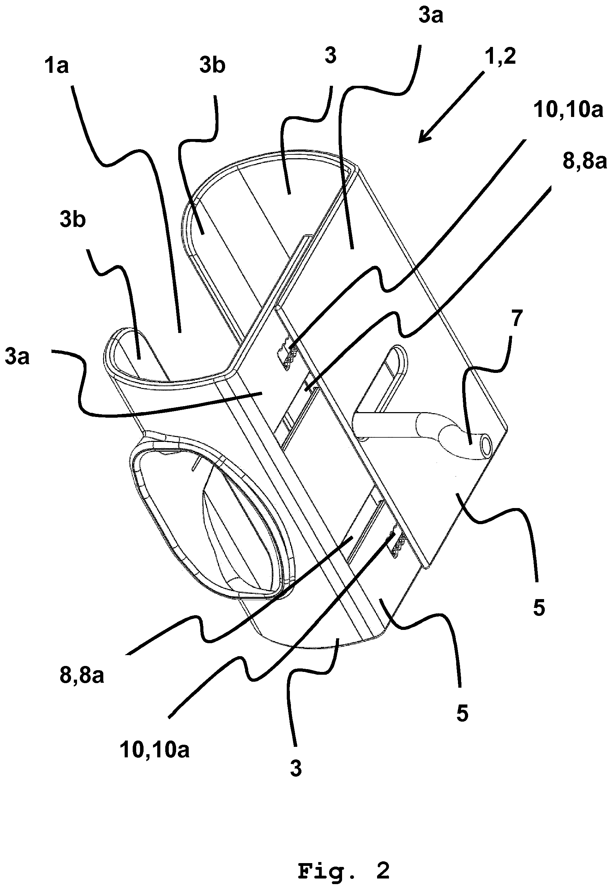

In Fig. 2 ist dieselbe Handmanschette 1 der erfindungsgemäßen Vorrichtung 2 dargestellt, wobei in Fig. 2 die Breite der Manschettenöffnung 1a wesentlich größer als in Fig. 1 gewählt ist. Dies erfolgt dadurch, dass die beiden Manschettenteile 3 so zueinander positioniert sind, dass der überlappende Abschnitt ihrer langen Schenkel 3a einen geringeren Überlapp aufweist als in Fig. 1. Dadurch wird die Breite der Manschettenöffnung 1a vergrößert.

Das Verbindungselement 4 der Handmanschette 1 mit den ebenen Abschnitten 5 weist zwei parallele Führungselemente 8 auf. Die beiden Führungselemente 8 werden durch eine Kulissenführung 8a gebildet, die ein gegenseitiges Verschieben der beiden Manschettenteile 3a entlang der Kulissenführung 8a ermöglicht. Dabei gleitet jeweils ein an einem Manschettenteil 3 befestigter Kulissenstein 8b in der Kulissenlaufbahn 8c, welche in dem anderen Manschettenteil 3 ausgebildet ist. Durch die parallele Ausrichtung der beiden Kulissenführungen 8a kann eine gleichmäßige und sichere, verkantungsarme Verschiebung der beiden Manschettenteile 3a gegeneinander erfolgen.

In Fig. 2 sind im Bereich der überlappenden, planen Abschnitte 5 neben den beiden parallel zueinander verlaufenden Kulissenführungen 8, 8a zusätzlich parallel verlaufende Fixierelemente 10, 10a ausgebildet. Die Fixierelemente stellen Rastverbindungen dar. Bei diesen greift mittels einer Formschlussverbindung 10a ein Formstein in eine korrespondierende rastende Ausnehmung ein und verhindert dadurch nach dem Eingriff eine weitere Verschiebung entlang der Kulissenführung 8a.

Um eine Verschiebung der Manschettenteile 3 gegeneinander zu ermöglichen, wird zuerst das Fixierelement 10, 10a aus dem Eingriff gebracht, um anschließend eine Verschiebung entlang der Kulissenführung 8a vorzunehmen. Ist die gewünschte Breite der Manschettenöffnung 1a erreicht, so wird das Fixierelement 10a genutzt, indem eine formschlüssige Verbindung durch das Fixierelement 10a erneut geschaffen wird, wodurch ein weiteres Verschieben der Manschettenteile gegeneinander aufgrund der rastenden Fixierung verhindert wird.

Durch diese Führung der erfindungsgemäßen Handmanschette 1 mit Hilfe der als Kulissenführung 8a ausgebildeten Führungselemente 8 in Verbindung mit den Fixierelementen 10 ist eine sehr verlässliche, sichere und einfache Handhabung der Handmanschette 1 bzw. der Vorrichtung 2 zur Behandlung des Karpaltunnelsyndroms geschaffen.

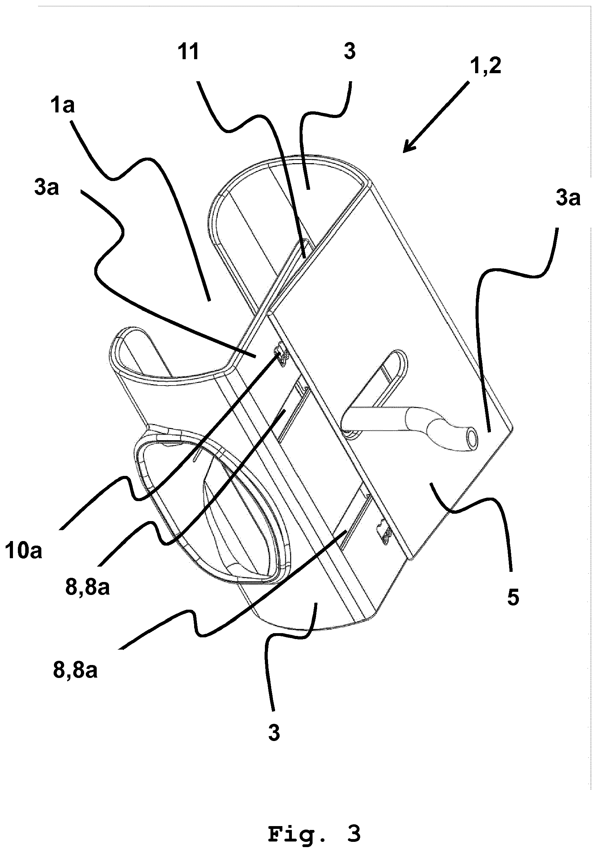

In Fig. 3 ist in einer perspektivischen Darstellung die Handmanschette 1 bzw. die Vorrichtung 2 während der Einstellung der Breite der Manschettenöffnung 1a dargestellt. Im Unterschied zu Fig. 2 ist in Fig. 3 das eine Manschettenteil 3 gegen das andere Manschettenteil 3 verkippt. Durch die Verkippung entsteht der sich über seine Länge öffnende Spalt 11 im Bereich des überlappenden Abschnitts 5. Durch diesen Spalt 11 ist das formschlüssige Element des formschlüssigen Fixierelementes 10a aus der rastenden Ausnehmung entnommen, wodurch die Beweglichkeit der Manschettenteile 3 im Rahmen der Bewegungsfreiheit der Kulissenführung 8a ermöglicht ist. Die relative Verschiebungsmöglichkeit der Manschettenteile 3 gegeneinander ist durch die Länge der Kulissenführungen 8a definiert. Der Anschlag dieser Kulissenführungen 8a beschränkt das Maß der Breite der Manschettenöffnung 1a. Dabei definiert es einerseits die Untergrenze der Breite der Manschettenöffnung 1a und andererseits zudem die maximale Breite der Manschettenöffnung 1a.

Durch die Ausbildung der Öffnungen in dem planen Abschnitt 5 zur Aufnahme des Schlauches 7 als Langloch 6a, das in seiner Länge der Länge der Kulissenführung 8a entspricht, wird gewährleistet, dass die Verschiebung nicht durch die Länge des Langloches 6a begrenzt ist bzw. dass durch die Verschiebung eine Beschädigung des Schlauches 7 bzw. ein Verschluss des Schlauches durch Abpressen durch die langen Schenkel 3a, die sich überlappen, gegeben ist. Dadurch ist die Funktionalität und Einstellbarkeit der Handmanschette 1 bzw. der Vorrichtung 2 in besonderem Maße gewährleistet.

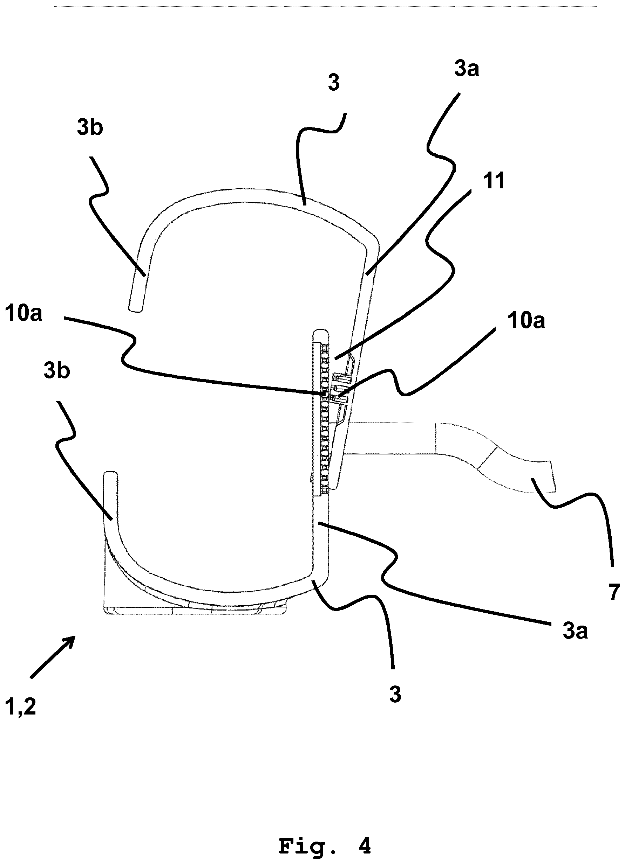

In Fig. 4 ist in einer Schnittdarstellung der Aufbau der Handmanschette 1 dargestellt. Die C-förmigen Manschettenteile 3 zeigen jeweils einen langen Schenkel 3a und einen kurzen Schenkel 3b. Die langen Schenkel 3a zeigen einen Überlappbereich (Abschnitt 5). Die Manschettenteile 3 sind gegeneinander verkippt, so dass sie im Überlappbereich einen Spalt 11 zeigen, der sich über seine Länge öffnet. In dem Spalt 11 ist das Fixierelement 10, 10a dargestellt. Es besteht aus zwei Teilen. Ein Teil zeigt eine charakteristische Formgebung und ist an dem oberen langen Schenkel 3a angeordnet. Das andere Teil zeigt eine Ausnehmung, die formschlüssig das erste Teil mit der charakteristischen Formgebung aufnehmen kann und aufgrund des Formschlusses ein Verschieben d. h. eine andere Positionierung der beiden Manschettenteile 3 gegeneinander verhindert.

In der in Fig. 4 dargestellten Situation ist das Fixierelement 10a nicht im Eingriff, so dass die Manschettenteile 3 durch die in Fig. 4 nicht dargestellten Kulissenführungen 8, 8a geleitet gegeneinander verschoben werden können.

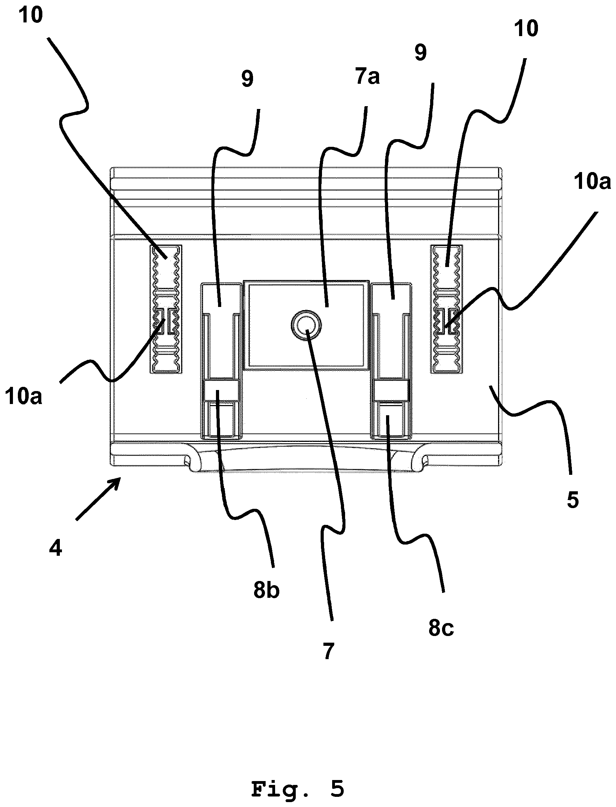

In Fig. 5 ist in einem horizontalen Schnitt im Bereich des überlappenden Abschnitts 5 das Verbindungselement 4 näher dargestellt. Es zeigt zwei parallele Kulissenführungen 8a mit den Kulissensteinen 8b, die ein Profil mit Hinterschnitt aufweisen und die in eine Kulissenlaufbahn 8c eingreifen, welche in ihrem Querprofil dem Kulissenstein 8b entsprechen und die Möglichkeit schaffen, entlang der Kulissenlaufbahn 8c sicher geführt verschoben zu werden.

Die Kulissenführung 8a zeigt an einem Ende einen Trennbereich 9, der kein Profil zeigt, das entsprechend der sonstigen Kulissenlaufbahn 8c einen Hinterschnitt zeigt. Vielmehr zeigt ein solcher Trennbereich 9 eine Öffnung, die in ihrem Querschnitt dem größten Querschnitt des Kulissensteins 8b entspricht. Das bedeutet, dass der Trennbereich in seiner Formgebung typisch der Form des Kulissensteins entspricht, wobei seine Größe gering größer gewählt ist. Durch diese Ausbildung des Trennbereichs 9 wird es möglich, den Kulissenstein 8b aus der Kulissenführung 8c zu entnehmen und dadurch die beiden Manschettenteile 3 voneinander zu trennen und damit voneinander zu lösen. Die durch dieses Verbindungselement 4 geschaffene, einstellbare Verbindung der Manschettenteile 3 der Handmanschette 1 kann damit voneinander gelöst werden und dadurch ein einzelnes selektives Reinigen, ein Warten der einzelnen Manschettenteile 3 bzw. ein Ersetzen durch ein anderes angepasstes Manschettenteil 3 erfolgen. Dadurch wird die Möglichkeit geschaffen, die Handhabung sehr sicher, verlässlich und dauerhaft zu gewährleisten.

Neben den beiden Führungselementen, die durch die Kulissensteine 8b und mit Kulissenlaufbahn 8c gebildet werden und parallel zueinander verlaufen, sind in dem Verbindungselement 4 zusätzlich zwei Fixierelemente 10a angeordnet. Diese zeigen eine längliche Ausnehmung, die mit einem gezackten Rand versehen ist. In diese gezackte Ausnehmung greift formschlüssig ein Formstein ein, der in der Eingriffssituation mit Hilfe des Formschlusses ein weiteres Verschieben der Manschettenteile 3 gegeneinander verhindert. Um dies zu erreichen, ist an einem Manschettenteil 3 der Formstein und an dem anderen Manschettenteil 3 die korrespondierende formschlüssige Ausnehmung angeordnet. Die Fixierung mit Hilfe der formschlüssigen Fixierelemente 10a erfolgt dabei im Wesentlichen in dem gleichen Längenmaß wie die Möglichkeit der Verschiebung entlang der Führungselemente, die hier als Kulissenführung 8a ausgebildet sind.

Im zentralen Bereich des Verbindungselementes 4 ist der Schlauch 7 mit einem Druckpolster 7a angeordnet und dargestellt. Durch das Aufblasen des Druckpolsters 7a zwischen dem Handrücken eines Patienten und dem Abschnitt 5 mit den langen Schenkeln 3a wird einerseits Druck auf die Handaußenseite gebildet und andererseits ein Druck in Richtung der Innenseite des langen Schenkels 3a eines Manschettenteils 3 der Handmanschette 1 gebildet.

Dadurch wird der eine lange Schenkel 3a gegen den anderen langen Schenkel 3a der Manschettenteile 3 im Bereich des Abschnittes 5 gepresst und die Fixierelemente 10a treten in Eingriff bzw. werden in Eingriff gehalten und dadurch vor einem ungewollten Öffnen gesichert.

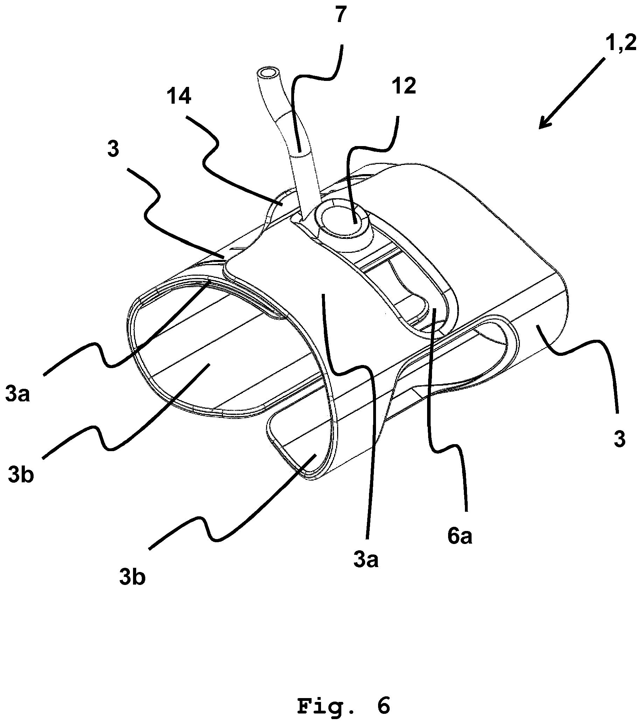

In Fig. 6 ist eine andere, beispielhafte Vorrichtung 2 zur Behandlung des Karpaltunnelsyndroms einer Person mit einer Handmanschette 1 mit zwei Manschettenteilen 3 dargestellt. Die zwei Manschettenteile 3 haben einen C-förmigen Querschnitt. Sie überlappen sich in einem Abschnitt 5, in dem sich zylinderförmig gekrümmte Flächen zumindest partiell überlappen. Der Überlapp erfolgt dabei im Bereich der langen Schenkel 3a der Manschettenteile 3. Der Überlapp kann durch gegenseitiges Verschieben, in der Art einer Verschwenkung, vergrößert oder verkleinert werden und dadurch die Breite der Handmanschette 2 vergrößert oder verkleinert werden.

Im Zentralbereich befindet sich wie in der Vorrichtung der Fig. 1 bis 5 ein Langloch 6a, durch das sich ein Schlauch 7 zur Befüllung oder Entleerung eines Druckpolsters 7a mit Hilfe eines Fluides erstreckt. Weiterhin erstreckt sich durch das Langloch 6a der Taster 12, der mit dem langen Schenkel 3a fest verbunden ist.

Die Ränder der Manschettenteile 3 sind insbesondere im Bereich des Langlochs 6a abgeschrägt ausgebildet. Im Bereich des Randes des äußeren C-förmigen Manschettenteils 3 im Bereich des Langlochs 6a im Zentralbereich ist ein Griff 14 vorgesehen, mit dessen Hilfe die beiden Manschettenteile 3 gegeneinander in ihrer Relativposition positioniert werden können. Zusätzlich hat der Griff 14 eine Schutzfunktion gegenüber dem Schlauch 7, so dass dieser insbesondere im Zusammenwirken mit dem Taster 12 vor ungewolltem Abknicken und Beschädigen geschützt ist.

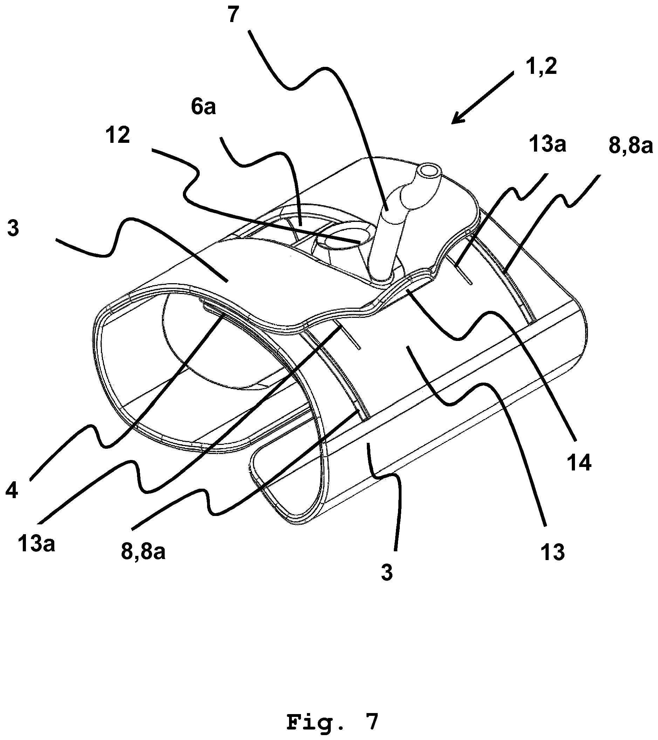

Die Vorrichtung 1 aus Fig. 6 ist in Fig. 7 in einer anderen Schrägansicht dargestellt. Sie zeigt den Aufbau des inneren Manschettenteils 3. Dessen langer Schenkel 3a zeigt zwei Führungselemente 8, die als Kulissenführung 8a ausgebildet sind. Zwischen den beiden, parallel verlaufenden Führungselementen 8, 8a sind zwei Schlitze 13a angeordnet, die zwei parallele, zu den Führungselementen 8, 8a parallel verlaufende Einschnitte darstellen.

Mit Hilfe der Schlitze 13a wird im Zentralbereich des langen Schenkels 3a eine federnde Lasche 13 gebildet. Auf der Oberseite der federnden Lasche 13 ist der Taster 12 angeordnet, der durch das Langloch 6a hindurchragt und dieses deutlich überragt. Durch den Rand des erhobenen Tasters 12 ist der Schlauch 7 geführt und durch die Erhebung des Tasters 12 sowie durch den Griff 14 geschützt. Der Überlapp der Manschettenteile 3 ist, wie in Fig. 6 und 7 dargestellt, gering, so dass die Breite der Handmanschette 1 groß ausfällt. Werden die Manschettenteile 3 nach einer Entriegelung der Fixierung durch Betätigung des Tasters 12 entlang der Führungselemente 8, 8a zusammengeschwenkt, so vergrößert sich der Überlapp und die Breite der Handmanschette 1 verringert sich. Dies wird dadurch erreicht, dass der Taster 12 in Richtung der federnden Lasche 13 gedrückt wird, dadurch die federnde Lasche nach innen ausgelenkt wird und die in Fig. 7 nicht dargestellten Fixierelemente 10, 10a aus dem Eingriff gebracht werden und dadurch eine Verschiebbarkeit der Manschettentele 3 gegeneinander ermöglicht wird. Um eine Fixierung der Relativposition der Manschettenteile 3 zueinander zu erreichen, wird der Taster 12 nicht mehr betätigt, so dass dieser mit der federnden Lasche 13 federnd nach außen in die Ruheposition zurückschwenkt und die Fixierelemente wieder in Eingriff treten. Durch diesen formschlüssigen Eingriff ist die Relativposition der Manschettenteile 3 fixiert.

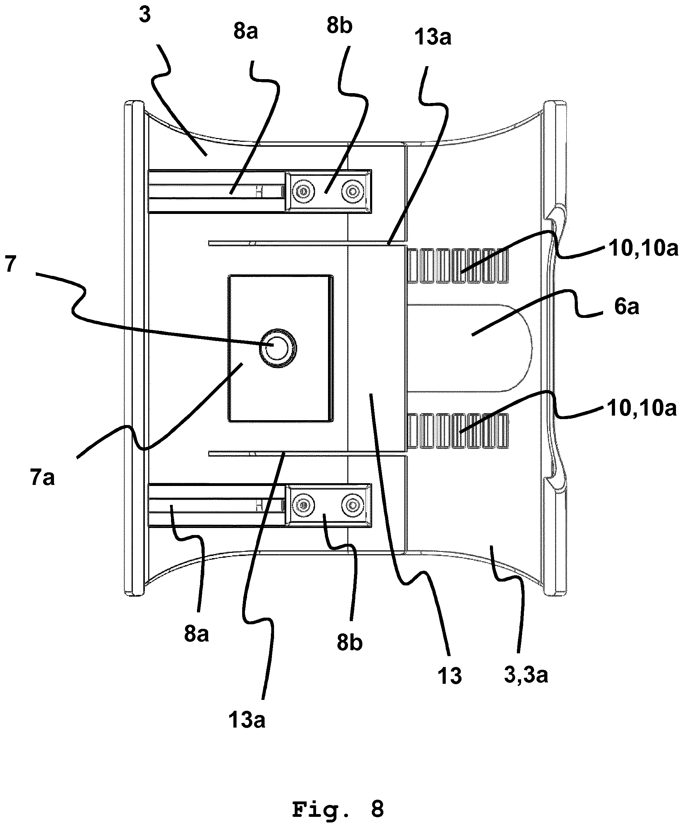

Die Unterseite der beiden langen Schenkel 3a ist in Fig. 8 dargestellt. Das linke, dargestellte Manschettenteil 3 mit dem langen Schenkel 3a zeigt zwei parallele Kulissenlaufbahnen 8c. Zwischen diesen befinden sich zwei parallel verlaufende Schlitze 13a, die die federnde Lasche 13 im Zentralbereich des linken Manschettenteils 3 bilden. Durch die einzig verbleibende stoffliche Verbindung der federnden Lasche 13 mit dem restlichen Manschettenteil 3 ist ein Federlager gebildet, um das die federnde Lasche 13 verschwenkt werden kann. Zentral auf der Unterseite der federnden Lasche 13 befindet sich das Druckpolster 7a, das mit Hilfe des Schlauches 7 befüllt oder entleert werden kann. Der Schlauch durchstößt eine im Wesentlichen kreisförmige Öffnung in der federnden Lasche 13 gefolgt von dem Langloch 6a in dem anderen Manschettenteil 3.

Auf der abgewandten, oberen Seite der federnden Lasche 13 befinden sich Rastelemente, die in die formschlüssigen Ausnehmungen 10a auf der Innenseite des anderen Manschettenteils 3 eingreifen können und gemeinsam das Fixierelement 10 bilden. Die Fixierelemente 10 werden durch den in Fig. 8 nicht dargestellten Taster 12 betätigt.

In die Kulissenlaufbahnen 8c greift je ein Kulissenstein 8b ein, der mit dem anderen Manschettenteil 3 fest verbunden ist und gemeinsam mit der zugordneten Kulissenlaufbahn eine Kulissenführung und damit ein Führungselement bildet, welches die Relativbewegung der Manschettenteile 3 zueinander definiert und beschränkt. Durch die Kulissensteine 8b und die zugeordnete Kulissenlaufbahn 8c ohne Trennbereich ist eine dauerhafte Verbindung der Manschettenteile 3 miteinander gewährleistet, wobei ihre Relativposition zueinander durch das Zusammenwirken der Führungselemente 8 und der Fixierelemente 10 definiert ist.

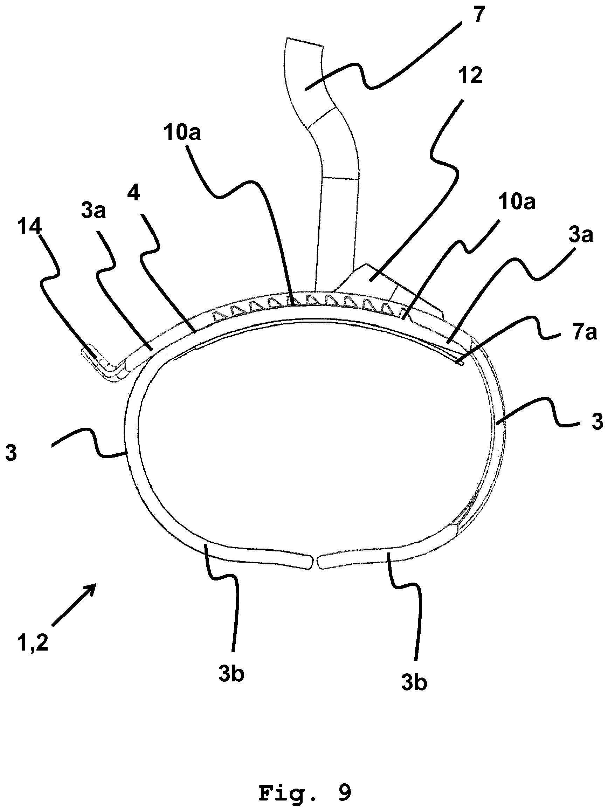

In Fig. 9 ist in einer Schnittdarstellung entlang des Fixierelementes 10, 10a die Vorrichtung 2 der Fig. 6 bis 8 in einem Zustand mit großem Überlapp und damit geringer Breite dargestellt. Der äußere lange Schenkel 3a des rechten C-förmigen Manschettenteils 3 zeigt an seinem Ende den Griff 14 und an seiner Innenseite mehrere formschlüssige Ausnehmungen 10a der Rastverbindungen, die gemeinsam mit dem formschlüssigen Rastelement 10a das als Rastverbindung ausgebildete Fixierelement 10 bilden. Die formschlüssigen Ausnehmungen 10a bzw. das Fixierelement 10a zeigen zwei unterschiedlich steile Flanken, wodurch ein Vergrößern des Überlapps der langen Schenkel 3a der Manschettenteile 3 erleichtert und ein Verkleinern des Überlapps erschwert wird. Durch die Betätigung des Tasters 12 wird die federnde Lasche 13 mit dem rastenden, formschlüssigen Fixierelement 10a, das radial nach außen die federnde Lasche 13 überragt, nach innen ausgelenkt, aus der formschlüssigen Ausnehmung entnommen und damit die Fixierung gelöst. Ein Verschwenken der Manschettenteile 3 kann nun leicht insbesondere unter Nutzung des Griffes 14 erfolgen.

Auf der Innenseite der federnden Lasche 13 ist das Druckpolster 7a angeordnet, das über den Schlauch 7 mit Fluid befüllt werden kann, so dass es in seiner Dicke zunimmt und den Zwischenraum zwischen der Hand des Patienten und der Handmanschette 1 ausfüllt und dadurch die als Rastverbindung ausgebildeten Fixierelemente 10 zusätzlich zu der Federkraft der federnden Lasche 13 gegen unbeabsichtigtes Lösen sichert.

Damit wird deutlich, dass die Verstellbarkeit und die Sicherheit von ungewolltem Verändern der Breite der Manschettenöffnung 1a in besonders vorteilhafter Weise gegeben ist und dadurch eine besonders effiziente und einfache Handhabung der erfindungsgemäßen Vorrichtung bzw. Handmanschette 1 gewährleistet ist.

### Bezugszeichenliste:

- 1: Handmanschette
- 1a: Manschettenöffnung
- 1b: Handöffnung
- 1c: Daumenöffnung
- 2: Vorrichtung zur Behandlung des Karpaltunnelsyndroms
- 3: Manschettenteil
- 3a: langer Schenkel des Manschettenteils
- 3b: kurzer Schenkel des Manschettenteils
- 4: Verbindungselement
- 5: überlappende Abschnitte
- 6: Öffnung; Loch
- 6a: Langloch
- 7: Schlauch
- 7a: Druckpolster
- 8: Führungselement
- 8a: Kulissenführung
- 8b: Kulissenstein
- 8c: Kulissenlaufbahn
- 9: Trennbereich
- 10: Fixierelement
- 10a: formschlüssiges Fixierelement, Rastverbindung
- 11: Spalt
- 12: Taster
- 13: Federnde Lasche
- 14: Griff

## Patentansprüche

1. Handmanschette (1) für eine Vorrichtung (2) zur Behandlung des Karpaltunnelsyndroms der Hand einer Person, die eine Palmarseite mit Thenar und Hypothenar Regionen und eine Dorsalseite gegenüber der Palmarseite aufweist,
die im Querschnitt als ein C-Profil ausgebildet ist, wobei die Enden des C-förmigen Profils eine Manschettenöffnung (1a) bilden,
wobei die Handmanschette (1) zwei oder mehr Manschettenteile (3) mit im Querschnitt C-förmigem Profil aufweist, die miteinander mittels wenigstens eines Verbindungselementes (4) die Handmanschette (1) bildend verbindbar sind und dass zur Anpassung der Breite der Manschettenöffnung (1a) Manschettenteile (3) gegeneinander bewegbar verbunden sind, **dadurch gekennzeichnet, dass** zur Anpassung der Breite der Manschettenöffnung (1a) Manschettenteile (3) gegeneinander verschiebbar und/oder verschwenkbar verbunden sind und
dass wenigstens ein Verbindungselement (4) Abschnitte (5) verbundener Manschettenteile (3) enthält, die sich wechselseitig überlappen, wobei die Abschnitte (5) überlappende Flächen (5) aufweisen.

2. Handmanschette (1) nach Anspruch 1, **dadurch gekennzeichnet, dass** Manschettenteile (3) zueinander rastend verschiebbar und/oder verschwenkbar ausgebildet sind.

3. Handmanschette (1) nach einem der vorstehenden Ansprüche, **dadurch gekennzeichnet, dass** wenigstens ein Verbindungselement (4) Abschnitte (5) verbundener Manschettenteile (3) enthält, die sich wechselseitig überlappen, wobei die Abschnitte (5) plane überlappende Flächen (5) aufweisen.

4. Handmanschette (1) nach einem der vorstehenden Ansprüche, **dadurch gekennzeichnet, dass** wenigstens ein Verbindungselement (4) mehrere Öffnungen (6, 6a) zur Aufnahme eines Schlauches (7) zur Befüllung oder Entnahme eines Fluides für ein in die Handmanschette (1) einbringbares Druckpolster (7a) aufweist, wobei wenigstens eine Öffnung (6a) als Langloch (6a) ausgebildet ist.

5. Handmanschette (1) nach einem der vorstehenden Ansprüche, **dadurch gekennzeichnet, dass** Manschettenteile (3) mit Führungselementen (8) zur Verschiebung und/oder Verschwenkung dieser Manschettenteile (3) gegeneinander verbunden sind, insbesondere mittels einer oder mehreren in einem Verbindungselement (4) angeordneten Nut-Feder-Verbindungen, Kulissenführungen (8a) und/oder Randführungen.

6. Handmanschette (1) nach Anspruch 5, **dadurch gekennzeichnet, dass** eine oder mehrere Kulissenführungen (8a) einen oder mehrere Kulissensteine (8b) mit Hinterschnitt und eine oder mehrere Kulissenlaufbahnen (8c) mit einem an den oder die Kulissensteine (8b) angepassten Profil aufweisen, wobei die Kulissenlaufbahnen (8c) einen Trennbereich (9) aufweisen, der eine Entnahme oder ein Einbringen des profilierten Kulissensteins (8b) aus der oder in die profilierte Kulissenlaufbahn (8c) ermöglicht.

7. Handmanschette (1) nach einem der vorstehenden Ansprüche, **dadurch gekennzeichnet, dass** Manschettenteile (3) eine Begrenzung der Verschiebung und/oder Verschwenkung insbesondere durch einen Anschlag oder durch ein Kulissenlaufbahnende einer Kulissenführung (8a) aufweisen.

8. Handmanschette (1) nach einem der vorstehenden Ansprüche, **dadurch gekennzeichnet, dass** das Verbindungselement (4) der gegeneinander verschiebbaren und/oder verschwenkbaren Manschettenteile (3) eine Skala zur Darstellung eines Maßes der Breite der Manschettenöffnung (1a) der Handmanschette (1) aufweist.

9. Handmanschette (1) nach einem der vorstehenden Ansprüche, **dadurch gekennzeichnet, dass** Manschettenteile (3) mittels einem oder mehrerer in einem Verbindungselement (4) angeordneter Fixierelemente (10, 10a) fixierbar verbunden sind.

10. Handmanschette (1) nach Anspruch 9, **dadurch gekennzeichnet, dass** ein oder mehrere Fixierelemente (10) durch eine Schraube-Mutter-Verbindung, Magnetverbindung, Klebeverbindung, Formschlussverbindung (10a) oder Klemmverbindung insbesondere mittels eines Kniehebelklemmelementes ausgebildet sind.

11. Handmanschette (1) nach Anspruch 10, **dadurch gekennzeichnet, dass** mehrere als Führungselemente (8) ausgebildete Kulissenführungen (8a) einen oder mehrere Kulissensteine (8b) mit Hinterschnitt und eine oder mehrere Kulissenlaufbahnen (8c) mit einem an den oder die Kulissensteine (8b) angepassten Profil vorgesehen sind, wobei im Zwischenbereich zwischen zwei Kulissenführungen (8a) wenigstens ein Fixierelement (10) angeordnet ist, wobei insbesondere im Zwischenbereich eine federnde Lasche (13) angeordnet ist
und wobei die Handmanschette (1) mit einem Taster (12) versehen ist, mittels dem die federnde Lausche (13) federnd ausgelenkt werden kann und dadurch das Fixierelement (10) gelöst und dadurch die Mannschettenteile (3) gegeneinander verschoben werden können.

12. Handmanschette (1) nach Anspruch 11, **dadurch gekennzeichnet, dass** der Taster (12) mit der federnden Lasche (13) verbunden ist und der Taster (12) so angeordnet und ausgebildet ist, dass der Taster (12) durch eine als Langloch (6a) ausgebildet Öffnung (6, 6a) betätigbar ist und insbesondere durch dieses hindurchragt.

13. Handmanschette (1) nach einem der vorstehenden Ansprüche, **dadurch gekennzeichnet, dass** Manschettenteile (3) lösbar verbunden sind.

14. Handmanschette (1) nach einem der vorstehenden Ansprüche, **dadurch gekennzeichnet, dass** Manschettenteile (3) einen C-förmigen Querschnitt mit unterschiedlich langen Schenkeln (3a, 3b) aufweisen
und dass Manschettenteile (3) einen Überlapp der längeren Schenkel (3a) verschiedener Manschettenteile (3) zur Bildung eines Verbindungselementes (4) aufweisen, das mit wenigstens einem Fixierelement (10) zur insbesondere lösbaren fixierenden Verbindung dieser verbundenen Manschettenteile (3) ausgebildet ist.

15. Handmanschette (1) nach einem der vorstehenden Ansprüche, **dadurch gekennzeichnet, dass** im Zentralbereich der Handmanschette (1) eine Verstärkung ausgebildet ist, die wenigstens ein Fixierelement (10), wenigstens ein Griffelement (14) zur Handhabung und/oder Positionierung der verbundenen Manschettenteile (3) gegeneinander, wenigstens ein Führungselement (8), wenigstens ein Rastelement und/oder wenigstens ein Loch (6) zeigt.

16. Vorrichtung (2) zur Behandlung des Karpaltunnelsyndroms der Hand einer Person, die eine Palmarseite mit Thenar und Hypothenar Regionen und eine Dorsalseite gegenüber der Palmarseite aufweist, mit einer Handmanschette (1) nach einem der vorstehenden Ansprüche und wenigstens einem Druckpolster (7a), das in der Handmanschette (1) eingebracht ist und das im Zusammenwirken mit den anderen Teilen der Vorrichtung (2) eine Vergrößerung des Karpaltunnels bewirken kann.

## Claims

1. Hand cuff (1) for a device (2) for treating carpal tunnel syndrome in a person's hand, which hand cuff has a palmar side having thenar and hypothenar regions and a dorsal side opposite the palmar side, and is formed as a C-profile in cross section, the ends of the C-shaped profile forming a cuff opening (1a), the hand cuff (1) having two or more cuff parts (3) that have a C-shaped profile in cross section and can be connected to one another by means of at least one connecting element (4) to form the hand cuff (1), and cuff parts (3) being connected so as to be movable relative to one another in order to adjust the width of the cuff opening (1a), **characterized in that** cuff parts (3) are connected so as to be displaceable and/or pivotable relative to one another in order to adjust the width of the cuff opening (1a), and **in that** at least one connecting element (4) contains portions (5) of connected, mutually overlapping cuff parts (3), the portions (5) having overlapping surfaces (5).

2. Hand cuff (1) according to claim 1, **characterized in that** cuff parts (3) are lockingly displaceable and/or pivotable relative to one another.

3. Hand cuff (1) according to either of the preceding claims, **characterized in that** at least one connecting element (4) contains portions (5) of connected, mutually overlapping cuff parts (3), the portions (5) having planar overlapping surfaces (5).

4. Hand cuff (1) according to any of the preceding claims, **characterized in that** at least one connecting element (4) has a plurality of openings (6, 6a) for receiving a hose (7) for loading or removing a fluid for a pressure pad (7a) that can be inserted into the hand cuff (1), at least one opening (6a) being formed as a slot (6a).

5. Hand cuff (1) according to any of the preceding claims, **characterized in that** cuff parts (3) having guide elements (8) for displacing and/or pivoting said cuff parts (3) relative to one another are connected, in particular by means of one or more tongue-and-groove connections, slide guides (8a) and/or edge guides arranged in a connecting element (4).

6. Hand cuff (1) according to claim 5, **characterized in that** one or more slide guides (8a) have one or more slide blocks (8b) having an undercut and one or more slide tracks (8c) having a profile adapted to the slide block(s) (8b), the slide tracks (8c) having a separation region (9) that allows the profiled slide block (8b) to be removed from or inserted into the profiled slide track (8c).

7. Hand cuff (1) according to any of the preceding claims, **characterized in that** the displacement and/or pivoting of cuff parts (3) is limited, in particular by means of a stop or a slide track end of a slide guide (8a).

8. Hand cuff (1) according to any of the preceding claims, **characterized in that** the connecting element (4) of the cuff parts (3) that are displaceable and/or pivotable relative to one another has a scale for displaying a measure for the width of the cuff opening (1a) of the hand cuff (1).

9. Hand cuff (1) according to any of the preceding claims, **characterized in that** cuff parts (3) are fixably connected by means of one or more fixing elements (10, 10a) arranged in a connecting element (4).

10. Hand cuff (1) according to claim 9, **characterized in that** one or more fixing elements (10) are formed by a bolt/nut connection, magnetic connection, adhesive connection, form-locking connection (10a) or clamping connection, in particular by means of a toggle clamp element.

11. Hand cuff (1) according to claim 10, **characterized in that** a plurality of slide guides (8a) designed as guide elements (8a) are provided, which slide guides comprise one or more slide blocks (8b) having an undercut and one or more slide tracks (8c) having a profile adapted to the slide block(s) (8b), at least one fixing element (10) being arranged in the intermediate region between two slide guides (8a), in particular a resilient tab (13) being arranged in the intermediate region, and the hand cuff (1) being provided with a pushbutton (12) by means of which the resilient tab (13) can be resiliently deflected, it thereby being possible to release the fixing element (10) and displace the cuff parts (3) relative to one another.

12. Hand cuff (1) according to claim 11, **characterized in that** the pushbutton (12) is connected to the resilient tab (13), and the pushbutton (12) is arranged and designed such that the pushbutton (12) can be operated through an opening (6, 6a) formed as a slot (6a) and in particular protrudes through said opening.

13. Hand cuff (1) according to any of the preceding claims, **characterized in that** cuff parts (3) are releasably connected.

14. Hand cuff (1) according to any of the preceding claims, **characterized in that** cuff parts (3) have a C-shaped cross section having legs (3a, 3b) of different lengths, and **in that** cuff parts (3) have an overlap between the longer legs (3a) of different cuff parts (3) so as to form a connecting element (4) designed having at least one fixing element (10) for in particular releasably fixedly connecting said connected cuff parts (3).

15. Hand cuff (1) according to any of the preceding claims, **characterized in that** a reinforcement is formed in the central region of the hand cuff (1), which reinforcement comprises at least one fixing element (10), at least one gripping element (14) for handling and/or positioning the connected cuff parts (3) relative to one another, at least one guide element (8), at least one locking element and/or at least one hole (6).

16. Device (2) for treating carpal tunnel syndrome in a person's hand, which device has a palmar side having thenar and hypothenar regions and a dorsal side opposite the palmar side, and comprises a hand cuff (1) according to any of the preceding claims and at least one pressure pad (7a), which is inserted in the hand cuff (1) and, in conjunction with the other parts of the device (2), can cause enlargement of the carpal tunnel.

## Revendications

1. Manchette (1) d'un dispositif (2) pour traiter le syndrome du canal carpien de la main d'une personne, qui présente un côté palmaire comportant des régions thénar et hypothénar et un côté dorsal opposé au côté palmaire, lequel côté dorsal présente une section transversale sous la forme de profil en C, les extrémités du profil en forme de C formant une ouverture de manchette (1a), la manchette (1) ayant deux ou plusieurs parties de manchette (3) présentant un profil en forme de C à section transversale, lesquelles parties de manchette peuvent être reliées l'une à l'autre au moyen d'au moins un élément d'assemblage (4) formant la manchette (1), et des parties de manchette (3) étant reliées les unes aux autres de manière mobile les unes par rapport aux autres, afin d'adapter la largeur de l'ouverture de manchette (1a), **caractérisée en ce que**, pour adapter la largeur de l'ouverture de manchette (1a), des parties de manchette (3) sont reliées les unes aux autres de manière à pouvoir se déplacer et/ou pivoter, et **en ce qu'**au moins un élément d'assemblage (4) comprend des sections (5) de parties de manchette reliées (3) qui se chevauchent mutuellement, les sections (5) présentant des surfaces se chevauchant (5).

2. Manchette (1) selon la revendication 1, **caractérisée en ce que** des parties de manchette (3) sont conçues de manière à pouvoir se déplacer et/ou pivoter les unes par rapport aux autres.

3. Manchette (1) selon l'une des revendications précédentes, **caractérisée en ce qu'**au moins un élément d'assemblage (4) contient des sections (5) de parties de manchon reliées (3) qui se chevauchent, les sections (5) présentant des surfaces planes qui se chevauchent (5).

4. Manchette (1) selon l'une des revendications précédentes, **caractérisée en ce qu'**au moins un élément d'assemblage (4) présente plusieurs ouvertures (6, 6a) permettant de recevoir un tube (7) destiné à remplir ou à extraire un fluide pour un coussin de pression (7a) qui peut être inséré dans la manchette (1), au moins une ouverture (6a) étant conçue sous la forme d'un trou oblong (6a).

5. Manchette (1) selon l'une des revendications précédentes, **caractérisée en ce que** des parties de manchette (3) comportant des éléments de guidage (8) permettant de déplacer et/ou de faire pivoter ces parties de manchette (3) sont reliées les uns par rapport aux autres, en particulier au moyen d'un ou de plusieurs assemblages à rainure et languette, de guides à coulisse (8a) et/ou de guides de bord disposés dans un élément d'assemblage (4).

6. Manchette (1) selon la revendication 5, **caractérisée en ce qu'**un ou plusieurs guides à coulisse (8a) comportent un ou plusieurs coulisseaux (8b) à contre-dépouille et un ou plusieurs chemins de roulement à coulisse (8c) présentant un profil adapté au ou aux coulisseaux (8b), les chemins de roulement à coulisse (8c) comportant une zone de séparation (9) qui permet de retirer le coulisseau profilé (8b) du chemin de roulement à coulisse profilé (8c) ou de l'insérer dans celui-ci.

7. Manchette (1) selon l'une des revendications précédentes, **caractérisée en ce que** des parties de manchette (3) présentent une limitation du déplacement et/ou du pivotement, en particulier moyennant une butée ou une extrémité de chemin de roulement à coulisse d'un guide à coulisse (8a).

8. Manchette (1) selon l'une des revendications précédentes, **caractérisée en ce que** l'élément d'assemblage (4) des parties de manchette (3) pouvant se déplacer et/ou pivoter les unes par rapport aux autres comporte une échelle destinée à représenter une mesure de la largeur de l'ouverture (1a) de la manchette (1).

9. Manchette (1) selon l'une des revendications précédentes, **caractérisée en ce que** des parties de manchette (3) sont reliées de manière fixe au moyen d'un ou de plusieurs éléments de fixation (10, 10a) disposés dans un élément d'assemblage (4).

10. Manchette (1) selon la revendication 9, **caractérisée en ce qu'**un ou plusieurs éléments de fixation (10) sont réalisés par un assemblage vis-écrou, un assemblage magnétique, un assemblage collé, un assemblage par complémentarité de forme (10a) ou un assemblage par serrage, en particulier au moyen d'un élément de serrage à levier à genouillère.

11. Manchette (1) selon la revendication 10, **caractérisée en ce qu'**il est prévu une pluralité de guides à coulisse (8a) conçus sous la forme d'éléments de guidage (8), un ou plusieurs coulisseaux (8b) à contre-dépouille et un ou plusieurs chemins de roulement à coulisse (8c) comportant un profil adapté au ou aux coulisseaux (8b), au moins un élément de fixation (10) étant disposé dans la zone intermédiaire entre deux guides à coulisse (8a), en particulier une languette élastique (13) étant disposée dans la zone intermédiaire, et la manchette (1) étant munie d'un bouton-poussoir (12) au moyen duquel la languette élastique (13) peut être déviée élastiquement, l'élément de fixation (10) pouvant ainsi être libéré, et les parties de manchette (3) pouvant ainsi être déplacées les unes par rapport aux autres.

12. Manchette (1) selon la revendication 11, **caractérisée** en ce le bouton-poussoir (12) est relié à la languette élastique (13) et le bouton-poussoir (12) est disposé et conçu de manière à ce que le bouton-poussoir (12) puisse être actionné par une ouverture (6, 6a) réalisée sous forme de trou oblong (6a), et en particulier de manière à ce qu'il traverse celui-ci.

13. Manchette (1) selon l'une des revendications précédentes, **caractérisée en ce que** des parties de manchette (3) sont reliées de manière amovible.

14. Manchette (1) selon l'une des revendications précédentes, **caractérisée en ce que** des parties de manchette (3) présentent une section transversale en forme de C à branches (3a, 3b) de différentes longueurs, et **en ce que** les parties de manchette (3) présentent un chevauchement des branches (3a) plus longues de différentes parties de manchette (3) pour former un élément d'assemblage (4) qui est réalisé avec au moins un élément de fixation (10) destiné en particulier à l'assemblage amovible des parties de manchette (3) reliées.

15. Manchette (1) selon l'une des revendications précédentes, **caractérisée en ce qu'**un renfort est réalisé dans la zone centrale de la manchette (1), lequel renfort comporte au moins un élément de fixation (10), au moins un élément de préhension (14) pour la manipulation et/ou le positionnement les uns par rapport aux autres des parties de manchette (3) reliées, au moins un élément de guidage (8), au moins un élément d'encliquetage et/ou au moins un trou (6).

16. Dispositif (2) pour traiter le syndrome du canal carpien de la main d'une personne, qui présente un côté palmaire comportant des régions thénar et hypothénar et un côté dorsal opposé au côté palmaire, comprenant une manchette (1) selon l'une des revendications précédentes, et au moins un coussin de pression (7a) qui est inséré dans la manchette (1) et qui peut induire un élargissement du canal carpien en coopération avec les autres parties du dispositif (2).
